# EUROPEAN PATENT SPECIFICATION

(11) **EP 1 626 279 B1**
(45) Date of publication and mention of the grant of the patent: **16.04.2008**
(21) Application number: 04025615.8
(22) Date of filing: 28.10.2004
(51) Int. Cl.: G01N 33/68

(54) **Method for diagnosing liver fibrosis**
Verfahren zur Diagnose von Leberfibrose
Procédé de diagnostic de fibrose hépatique

(30) Priority: 12.08.2004 EP 04019133
(43) Date of publication of application: 15.02.2006
(73) Proprietor: Roche Diagnostics GmbH, 68305 Mannheim (DE); F. Hoffmann-La Roche AG, 4070 Basel (CH); UNIVERSITE D'ANGERS, 49035 Angers (FR)
(72) Inventor: Wienhues-Thelen, Ursula, Dr., 82152 Krailling (DE); Huedig, Hendrik, Dr., 82377 Penzberg (DE); Calès, Paul, Prof., 49933 Angers Cedex 9 (FR)

(56) References cited:
- WO-A-03/073822
- MORRISON ELIZABETH D ET AL: "Serum ferritin level predicts advanced hepatic fibrosis among U.S. patients with phenotypic hemochromatosis." ANNALS OF INTERNAL MEDICINE. 15 APR 2003, vol. 138, no. 8, 15 April 2003 (2003-04-15), pages 627-633, XP002350226 ISSN: 1539-3704
- DATABASE MEDLINE [Online] US NATIONAL LIBRARY OF MEDICINE (NLM), BETHESDA, MD, US; May 2003 (2003-05), HUANG CONG-WU ET AL: "[Relationship of serum iron and ferritin with the indicators for hepatic fibrosis]" XP002350228 Database accession no. NLM12754132 & DI YI JUN YI DA XUE XUE BAO = ACADEMIC JOURNAL OF THE FIRST MEDICAL COLLEGE OF PLA. MAY 2003, vol. 23, no. 5, May 2003 (2003-05), pages 466-468, ISSN: 1000-2588
- METWALLY MOHAMED A ET AL: "Clinical significance of hepatic iron deposition and serum iron values in patients with chronic hepatitis C infection." THE AMERICAN JOURNAL OF GASTROENTEROLOGY. FEB 2004, vol. 99, no. 2, February 2004 (2004-02), pages 286-291, XP002350225 ISSN: 0002-9270
- DATABASE WPI Section Ch, Week 200028 Derwent Publications Ltd., London, GB; Class B04, AN 2000-327185 XP002350230 & RU 2 132 071 C1 (NEMOV V V) 20 June 1999 (1999-06-20)
- D'SOUZA R F C ET AL: "Relationship between serum ferritin, hepatic iron staining, diabetes mellitus and fibrosis progression in patients with chronic hepatitis C." ALIMENTARY PHARMACOLOGY & THERAPEUTICS. 1 MAR 2005, vol. 21, no. 5, 1 March 2005 (2005-03-01), pages 519-524, XP002350227 ISSN: 0269-2813

## Description

### Field of the invention

The present invention relates to the fields of hepatology and liver fibrosis. In particular it relates to a panel of serological markers that can be used for diagnosing liver fibrosis, in particular used for diagnosing liver fibrosis due to chronic HCV infection. These markers can be used for monitoring therapeutic treatment of liver fibrosis.

### Background of the invention

Fibrotic liver disease ranks as the eighth most common cause of mortality worldwide, accounting for 1.3 million deaths annually (Murray and Lopez, 1997, Lancet 349,1269-1276). The cellular mechanisms of fibrosis are complex. In response to liver injury, for example caused by chronic hepatitis C virus (HCV) infection, hepatitis B virus (HBV) infection, alcoholic or fatty liver disease, drug-induced liver disease or primary biliary cirrhosis, normally quiescent hepatic stellate cells are activated into proliferating myofibroblasts. These cells produce extracellular matrix proteins and release tissue inhibitors of metalloproteinases which bind and inactivate metalloproteinases responsible for scar degradation. As a result, fibrosis and scar may accumulate through increased production of tissue and proteins like collagen and decreased degradation of these compounds so that the function of liver is impaired (McHutchinson 2004, CME Newsletter Tx Reporter Gastroenterology, 2-4).

While hepatic fibrosis is a reversible process resulting in the accumulation of extracellular matrix, liver cirrhosis is an irreversible process which is characterized by thick bands of matrix which completely encircle the parenchyma to form nodules. If left untreated, liver fibrosis may lead to cirrhosis, maybe cancer. For these reasons timely and accurate diagnosis of liver fibrosis is essential to effective medical treatment.

Currently liver biopsy is still considered as the so-called gold standard for assessment of fibrosis and inflammation. Liver biopsy is recommended to grade and stage the disease, confirm the diagnosis and establish a baseline against which to document improvement or disease progression, aid in determining prognosis and need for therapy (McHutchinson, see above; for review see Gebo et al. 2002 Hepatology 36, 161-172).

There exist numerous histologic grading systems that have been used to semiquantify the degree of hepatic fibrosis and inflammation in patients with chronic hepatitis C. One of the mostly used grading systems is the METAVIR system (Bedossa et al., 1994, Hepatology, 20, 15-20). METAVIR classifies hepatic fibrosis in 5 stages ranging from F0 to F4. F0 means no fibrosis, F1 corresponds to mild fibrosis (portal fibrosis without septa). The moderate to severe fibrosis classifies as F2 to F4 (F2: few septa, F3: numerous septa without cirrhosis), stage F4 corresponding to the ultimate stage of cirrhosis. Fibrosis is regarded as clinically significant starting from F ≥ 2.

But there are several disadvantages in applying liver biopsy for diagnosing and staging fibrosis. Liver fibrosis is subject to sampling error so that the small portion of sample might not reflect the real situation in the whole liver. As such it is not an accurate marker of the dynamic process of constant degradation. Further pathologists often do not agree in their readings of histologic samples where inter- and intra-observer variability occurs in 10 to 20 % of biopsies (Cadranel et al 2000, Hepatology 32, 477-481).

Liver biopsy is an invasive and painful procedure for the patient. It is also associated with a risk of hemorrhage and other complications after the sampling. Moreover and partly due to expected complications followed by hospitalization of the patient it is a costly procedure.

Hepatic fibrosis is the principal complication of chronic HCV infection leading to the development of cirrhosis and decompensated liver disease. Directed investigation examining the development and progression of fibrosis is, therefore, essential for effective management of these patients. Evaluation of progressive fibrosis will best be accomplished with noninvasive tests capable of discriminating intermediate stages of fibrosis. A variety of single markers and marker panel algorithms have been published, but no powerful single biomarker or biomarker score is currently available that allows a reliable prediction of fibrosis (Diagnostic Accuracy > 80 %). Further research into the development of noninvasive dynamic measures of hepatic fibrosis is strongly encouraged by the National Institutes of Health Consensus Development Conference in 2002. In particular the studies on alternatives to liver biopsy should provide enough details about the biopsy methods (average size of biopsy samples; histologically well characterized qualifying panel) to convince readers of the adequacy of reference standard. Liver biopsy is strongly dependent on optimized performance criteria and may lead to misclassification of histological stages due to interobserver variability and too small sample sizes (< 10 mm).

There has been a wide search for biochemical or serological markers which reflect fibrotic processes in liver disease and which can serve as a surrogate for liver biopsy. In the last years a couple of non-invasive or minimally invasive biochemical and serological markers have been investigated to assist in diagnosing liver diseases. In particular combinations of markers have been used to categorize patients according to their stage or degree of fibrosis.

US 6,631,330 discloses the use of a combination of at least 4 biochemical markers selected from the group consisting of α-2-macroglobulin, aspartate aminotransferase, γ-glutamyl transpeptidase, γ-globulin, total bilirubin, albumin, α1-globulin, α2-globulin, haptoglobin, β-globulin, apoA1, IL-10, TGF- β1, apoA2 and ApoB. The obtained values of 4 of these markers are mathematically combined to determine the presence of liver fibrosis. With this marker panel a diagnostic accuracy of about 80 per cent can be obtained.

The international patent application WO 2003/073822 describes a method for diagnosing the presence or severity of liver fibrosis in a patient. This method uses the combination of at least three markers which are α-2-macroglobulin, hyaluronic acid and tissue inhibitor of metalloproteinase 1 (TIMP-1). With this method a diagnostic accuracy of about 80 per cent (McHutchinson, 2004, see above) can be obtained.

There is a need to develop a non- or minimally invasive method to reach a higher diagnostic accuracy in determining liver fibrosis and classify and discriminate between different stages of fibrosis in a more reliable way than so far known in the state of the art so that monitoring of clinical development of fibrosis during therapeutic treatment is possible. Moreover such a method should be suitable for serial testing on automatic analyzers.

### Description of the invention

The problem is solved by a method according to the current invention. This method for the detection of the presence and/or the severity of a liver disease in a sample from a patient comprises the steps as follows:
a) measuring TIMP-1 (Tissue Inhibitor of Metalloproteinase I) in said sample
b) measuring ferritin in said sample
c) measuring one additional parameter selected from the group consisting of A2M (alpha-2-macroglobulin), PI (prothrombin index) in said sample
d) diagnosing the presence and/or severity of a liver disease based on the presence or measured levels of TIMP-1, ferritin and the parameter measured according to step c).

The present invention permits a reliable differentiation between F0/F1 fibrosis from F2/F3/F4 stages. Moreover therapeutic monitoring as a control of medical treatment of liver diseases can be carried out by the method of the invention.

The method of the current invention is highly correlating with well characterized Metavir stages of hepatic fibrosis. A special advantage of the method of the current invention in comparison to state of the art methods is the usage of a qualifying panel to minimize errors of misclassification of pathological observation and of statistical models.

The method of the invention comprises a noninvasive method correlating very closely with the severity of fibrosis as determined by several methods: liver biopsy and further methods such as the determination of the area of fibrosis.

-The method of the current invention is based on a statistically relevant cohort of specimens of patients with well characterized liver fibrosis, covering the total range of Metavir stages and of specimens of subjects without hepatic fibrosis due to histological findings (Metavir score: 0). The initial selection criteria of specimens is the Metavir score. This reference is confirmed in a double evaluation and in an optimized way using specimens with sizes larger than 15 mm.

The method of the current invention allows a reliable prediction of fibrosis with a diagnostic accuracy (DA) of at least 82 %, preferably at least 84 %. Since even the reference standard is no gold standard of hepatic fibrosis with respect to optional misclassification of fibrosis stages and further leads to pain and health risk to the patient the method of the present invention represents an alternative to biopsy.

The method allows the investigation of the development and progression of fibrosis providing an effective management of patients with chronic HCV. Disease monitoring of patients with chronic HCV may be performed in a short time interval in comparison to biopsy. The method allows monitoring the success of antifibrotic therapy.

The method also allows the investigation of the development and progression of fibrosis in subjects with chronic hepatic injury. This is a relatively common disorder with minimal symptoms, yet with long term risk of significant morbidity and mortality, which is defined pathologically by ongoing hepatic necrosis and inflammation in the liver, often accompanied by fibrosis. HCV is the most common form of chronic hepatic injury. The method can be applied to further forms of chronic hepatic injury: alcoholic steatohepatitis (ASH), alcoholic fatty liver disease (AFLD), non-alcoholic steatohepatitis (NASH) or non-alcoholic fatty liver disease (NAFLD).The methods of the invention can be used to monitor the severity of NASH and NAFLD. They can be used to diagnose liver fibrosis in an individual with viral hepatitis such as hepatitis A, B, C or D virus or a human immunodeficiency virus (HIV), chronic persistent or chronic active hepatitis, autoimmune liver disease, such as autoimmune hepatitis und drug-induced liver disease; primary biliary cirrhosis, primary sclerosing cholangitis, biliary atresia, liver disease resulting from medical treatment or a congenital liver disease. The invention can be used for monitoring of treatment with a drug with the risk of liver disease. The methods can be used for diagnosing the presence or severity of fibrosis and for monitoring fibrosis, wherein fibrosis is associated with a variety of fibrotic disorders not limited to the liver: puhnonary fibrosis, kidney fibrosis, prostate fibrosis and breast fibrosis ad further fibrosis in another disorder.

Combinations of parameters are TIMP-1, ferritin and A2M (also named SNIFF 3 a, SNIFF being the French abbreviation for score non-invasif de fibrose du foie; in English: non-invasive score of liver fibrosis) with a diagnostic accuracy of 82.6 %; TIMP-1, ferritin and PI (SNIFF 3) with a diagnostic accuracy of 84 %; and TIMP-1, ferritin, PI, PLT, urea, age with a diagnostic accuracy of 84.7 %. These preferred combinations can also be seen on table 3.

In the sense of the present invention the specific terms and expressions should be understood as follows:

Diagnostic accuracy (DA) is the accuracy of the test itself. This means the percentage of all tests that are truly positive or truly negative. The higher the diagnostic accuracy the more reliable are the results of the test. DA is calculated as the sum of true positives and true negatives divided by the total number of sample results and is affected by the prevalence of fibrosis in the population analyzed.

Cut-off value is the arithmetic calculated concentration of a single biomarker or of a combination of several biomarkers for the discrimination of healthy and disease state. In the understanding of the invention cut-off means a score of 0.5. If this value is above or equal to 0.5 (≥0.5) this means that the Metavir stage F2 is reached for the distinction between no or mild fibrosis (Metavir stages F0 or F1) and clinically significant fibrosis CSF (Metavir stages F2, F3, F4).

-Positive predictive value (PPV) is the percentage of positive tests that are truly positive.

Negative predictive value (NPV) means the percentage of negative tests that are truly negative.

Score means an arithmetic combination of several biomarkers associated with fibrosis. In particular, the score used herein has a range between 0 (minimal fibrosis) and 1 (CSF: clinically significant fibrosis).

AUROC means area under the receiver operator characteristics curve. In these curves, sensitivity is plotted against the reciprocal of specificity. An area under the ROC curve of 1.00 would indicate an ideal of 100 per cent sensitivity and 100 per cent specificity. The larger the slope at the beginning of the curve the better is the relation between sensitivity and specificity of a test.

Sensitivity is the probability of a positive test result in a patient with a disease or risk factor or other health condition.

Specificity is the probability of a negative test result in a patient who does not have the disease.

TIMP-1 (Tissue Inhibitor of Metalloproteinase I) is a 184 amino acid sialoglycoprotein with a molecular weight of 28.5 kDa (see e.g. Murphy et al Biochem J. 1981, 195,167-170) which inhibits metalloproteinases like interstitial collagenase MMP-1 or stromelysin or gelatinase B. In the understanding of the current invention the term TIMP-1 encompasses a protein with significant structural homology to human TIMP-1 inhibiting the proteolytic activity of metalloproteinases. The presence of human TIMP-1 can be detected by using antibodies that specifically detect epitopes of TIMP-1. TIMP-1 may also be determined by detection of related nucleic acids such as the corresponding mRNA.

Ferritin is a macromolecule with a molecular weight of at least 440 kD, depending on the iron content, and consists of a protein shell (apoferritin) of 24 subunits and an iron core containing an average of approximately 2500 Fe ³⁺ ions (in liver and spleen ferritin). Ferritin tends to form oligomers. At least 20 isoferritins can be distinguished with the aid of ioselectric focusing. This microheterogeneity is due to differences in the contents of the acidic H and weakly basic L subunits. The basic isoferritins are responsible for the long-term iron storage function, and are mainly found in liver, spleen and bone marrow.

The determination of ferritin is a suitable method for ascertaining the iron metabolism situation. Determination of ferritin at the beginning of therapy provides a representative measure of the body's reserves. Clinically, a threshold value of about 20 ng/ml has proved useful in the detection of prelatent iron deficiency. This value provides a reliable indication of exhaustion of the iron reserves that can be mobilized for hemoglobin synthesis. Latent iron deficiency is defined as a fall below the 12 ng/ml threshold. For manifestation of iron overloading in the body a threshold value above 400 ng/ml is regarded as useful.

For the detection of ferritin a classical sandwich immunoassay may be used in which two antibodies specific for ferritin are used to form a sandwich complex in the assay. One of the antibodies binds to a solid phase and the other antibody carries a label the signal of which is used a detection means for the presence of ferritin.

PI (prothrombin index) is useful to detect interferences in the coagulation system and can be determined by adding thromboplastine to the plasma sample and measuring the time of coagulation in seconds (so-called Quick-time).This value is correlated to an international normalized ratio that contains a correction factor that takes into account the sensitivity of the thromboplastine used.

A2M (α-2-Macroglobulin) is a conserved protein, highly abundant in plasma that serves as a protease binding protein to clear active proteases from tissue fluids. A2M does not inactivate the catalytic activity of a protease but acts by physical entrapment of the target protease by folding around the protease. A protease entrapped by A2M is thus sterically prevented from cleaving its substrate proteins. In the sense of the invention A2M may be detected by an immunological assay using specific antibodies according to test formats known to a person skilled in the art. A2M may also be determined by detection of related nucleic acids such as the corresponding mRNA

Described are also additional biochemical or clinical parameters . Additional biochemical parameter may be any parameter directly or indirectly associated with metabolism or structure of the liver as for example urea, GGT (gamma-glutamyltransferase), hyaluronate, AST (aspartate amino transferase), MMP-2 (matrix metalloproteinase-2), ALT (alanine aminotransferase), PIIINP (N-terminal propeptide of type III procollagen), bilirubin, haptoglobin, ApoA1, PLT (number of platelets). Also hepcidin or adiponectin may be determined.

Hepcidin is a hepatic protein, originally identified as a circulating antimicrobial peptide. It is central player in the communication of body iron stores to the intestinal absorptive cells. Adiponectin is secreted by the adipocytes and circulated at relatively high systemic concentrations to influence metabolic function. Reduced serum adiponectin levels indicate an increased risk of diseases for example severity of nonalcoholic fatty liver disease (NAFLD) or nonalcoholic steatohepatitis (NASH).

Additional clinical parameters are also disclosed such as age, sex, weight, nutritional habits of the patient.

Urea, GGT (gamma-glutamyltransferase), hyaluronate, AST (aspartate amino transferase) and ALT (alanine amino transferase), MMP-2, PIIINP bilirubin, haptoglobin, ApoA1, hepeidin and adiponectin are determined by commercially available test kits by immunological or photometrical methods known to a person skilled in the art. Where applicable also hybridization techniques for the detection of nucleic acids that are specific for an analyte or parameter (such as the corresponding mRNA) may be used for determination of a parameter.

PLT (number of blood platelets) is the number of blood platelets and is determined by counting the platelets using a commercially available counter.

The invention makes use of the determination of a plurality of parameters. Therefore the detection of those biochemical and serological parameters of the invention that may be carried out in test formats using a solid phase is preferably carried out on miniaturized array-based test systems as described in US 2003/0017616 or WO 99/67643. These test systems have multiple spatially defined test areas each of which can be used to detect a single specific analyte or parameter. Thus a plurality of analytes can be detected in a single test run.

The term defined test areas on a solid phase is understood to mean that the test areas comprise defined regions of the solid phase which are preferably spatially separated from other test areas by inert regions. The defined test areas preferably have a diameter of 10 µm to 1 cm and particularly preferably 10 µm to 5 mm. Miniaturized test areas with a diameter of 10 µm to 2 mm are most preferred. Solid phases with several test areas are preferred which are also referred to as array systems. Such array systems are for example described in Ekins and Chu (Clin. Chem. 37, 1995, 1955-1967) and in U.S. patents nos. 5,432,099, 5,516,635 and 5,126,276. As mentioned before, an advantage of array systems is that several analyte and control determinations can be carried out simultaneously on one sample. The use of control areas to detect unspecific binding and/or interfering samples can considerably improve the reliability of the results especially with miniaturized array test systems.

In the current invention the detection of TIMP-1 Ferritin, and further A2M or Pi could for example be performed simultaneously by using such an array-based test system.

According to the invention the solid phase is any conventional support for detection methods, preferably a non-porous support, e.g. a support with a plastic, glass, metal or metal oxide surface. Porous supports such as test strips are also suitable. Spatially discrete regions (test areas) are located on this support. Immobilized solid phase receptors are applied to these test areas. The solid phase receptors are immobilized by known methods, e.g. by direct adsorptive binding, by covalent coupling or by coupling via high affinity binding pairs, e.g. streptavidin(or avidin)/biotin, antigen/antibody or sugar/lectin. The presence or/and the amount of the analyte in a sample can be determined by specific binding of components from the detection medium, e.g. of the analyte to be determined or of an analyte analogue to the solid phase receptor.

The detection of the analyte and - where appropriate - the detection of the presence of interfering reactions is achieved in the method according to the invention in a known manner by using suitable marker groups, e.g. fluorescent marker groups. Alternatively with suitable solid phases it is possible to also detect the interaction of components of the detection medium with the test and optionally control areas by determining the layer thickness of the respective area, e.g. by plasmon resonance spectroscopy.

With array systems in which several analytes from a sample are detected simultaneously, it is preferable to use a universal marker group which enables a simultaneous detection of several different analytes to different test areas. An example of such universal marker groups are marker groups which carry a receptor that can specifically interact with a complementary receptor on a test reagent, e.g. a soluble receptor for an analyte to be determined or for an analyte analogue (like antibody/antigen or streptavidin/biotin etc.).

The term sample means a biological specimen that contains or allegedly contains at least one of the markers according to the invention. For example as a sample blood, serum, plasma, urine, saliva, synovial fluid or liver tissue may be used. Fluid samples may be diluted prior to analysis if required.

To obtain a result assisting in diagnosing the disease mathematical algorithms are used known to a person skilled in the art. The obtained data is combined and evaluated by statistical methods like logistic binary regression, resulting in scores.

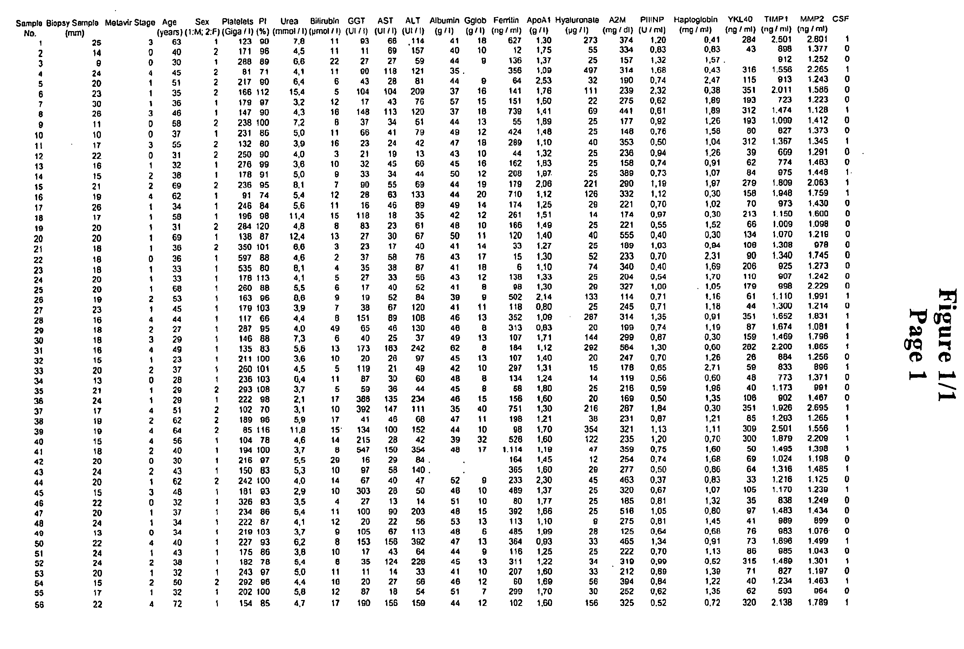

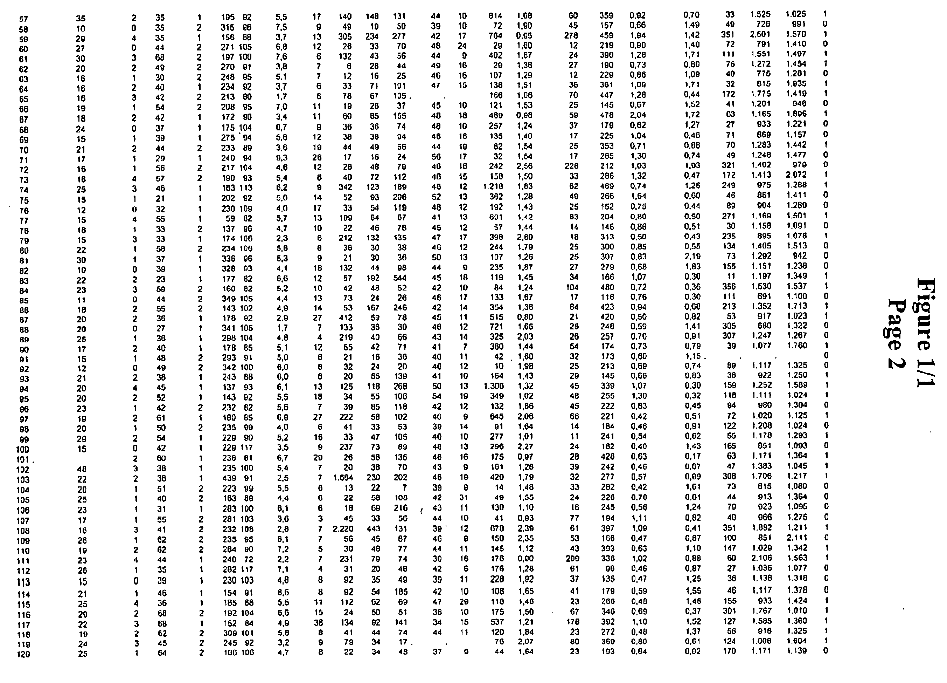

Figure 1 shows raw data as measured on 120 patient suffering from infection with HCV.

### Comparative Example

Commercially available test kits were used and all tests were performed according to the instructions given by the manufacturers as listed below.

**Table 1**

| Biomarker | Method | Provider |
|---|---|---|
| AST, ALT | Clinical Blood Chemistry | Roche Diagnostics GmbH Mannheim, Germany |
| GGT | Clinical Blood Chemistry | Roche Diagnostics GmbH Mannheim, Germany |
| Bilirubin | Clinical Blood Chemistry | Roche Diagnostics GmbH Mannheim, Germany |
| Urea | Clinical Blood Chemistry | Roche Diagnostics GmbH Mannheim, Germany |
| A2M | Nephelometry | Dade Behring Marburg GmbH |
| Apo A1 | Nephelometry | Dade Behring Marburg GmbH |
| Platelets | Platelet count | Bayer Diagnostics |
| PI | Coagulation Time | Diagnostica Stago |
| Hyaluronate | Elisa | Corgenix Inc. |
| PIIINP | RIA | Cis Bio International |
| YKL-40 | Elisa | Quidel Corporation |
| TIMP1 | Elisa | Amersham Pharmacia |
| MMP2 | Elisa | Amersham Pharmacia |

Figure 1 shows raw data as measured on samples of 120 patient suffering from infection with HCV. To obtain the data the test kits listed above were used.

In table 2 the diagnostic accuracy and the AUROC values are listed. It can be seen each single marker has got a DA below 80 %.

**Table 2**

| **Biomarker** | **Diagnostic Accuracy** | | **Correlation** | | **AUROC** | |
|---|---|---|---|---|---|---|
| | **%** | **p** | **r** | **p** | **c** | **p** |
| A2M | 76.7 | < 10⁻⁴ | 0.523 | < 10⁻⁴ | 0.800 | < 10⁻⁴ |
| TIMP 1 | 72.3 | < 10⁻⁴ | 0.663 | < 10⁻⁴ | 0.813 | < 10⁻⁴ |
| Ferritin | 71.7 | < 10⁻⁴ | 0.433 | < 10⁻⁴ | 0.771 | < 10⁻⁴ |
| HA | 71.7 | 0.002 | 0.561 | < 10⁻⁴ | 0.762 | < 10⁻⁴ |
| Platelets | 70.8 | < 10⁻⁴ | -0.523 | < 10⁻⁴ | 0.259 | < 10⁻⁴ |
| AST | 69.2 | < 10⁻⁴ | 0.444 | < 10⁻⁴ | 0.782 | < 10⁻⁴ |
| Prothrombin index | 69.2 | < 10⁻⁴ | -0.444 | < 10⁻⁴ | 0.265 | < 10⁻⁴ |
| GGT | 67.5 | 0.002 | 0.229 | 0.012 | 0.721 | < 10⁻⁴ |
| MMP2 | 67.2 | < 10⁻⁴ | 0.451 | < 10⁻⁴ | 0.711 | < 10⁻⁴ |
| ALT | 66.7 | 0.002 | 0.311 | 0.001 | 0.696 | < 10⁻⁴ |
| YKL 40 | 65.3 | 0.001 | 0.480 | < 10⁻⁴ | 0.661 | 0.002 |
| Age | 62.5 | 0.001 | 0.345 | < 10⁻⁴ | 0.706 | < 10⁻⁴ |
| P3P | 62.5 | 0.02 | 0.337 | < 10⁻⁴ | 0.626 | 0.019 |
| Bilirubin | 61.7 | 0.02 | 0.107 | 0.247 | 0.628 | 0.017 |
| Haptoglobin | 61.7 | 0.01 | -0.285 | 0.002 | 0.356 | 0.007 |
| ApoA1 | 60.0 | 0.03 | -0.229 | 0.012 | 0.361 | 0.009 |
| Sex | 53.3 | 0.37 | - | - | - | - |
| Urea | 51.7 | 0.28 | -0.058 | 0.527 | 0.470 | 0.572 |

Table 3 shows a comparison of DA /AUROC with state of the art methods. The methods of the current invention were shown to have superior diagnostic accuracy of clinically significant fibrosis by binary logistic regression in comparison with the methods of US 6,631,330 and WO 2003/073822.

**Table 3**

| **Method** | **Selected Markers** | **n var** | **n Pts** | **R²** | **DA** | **AUROC** |
|---|---|---|---|---|---|---|
| | PLT, PI, Urea, Fer, age, TIMP | 6 | 118 | 0.689 | 84.7 | |
| | TEMP, Fer, PI | 3 | 119 | 0.629 | 84.0 | 0.904 |
| | TIMP, Fer, A2M | 3 | 118 | | 82.6 | 0.886 |
| WO 2003/073822 | TIMP, HA, A2M | 3 | 118 | | 80.7 | 0.898 |
| US 6,631,330(Fibrotest) | A2M, age, Hapto, Apo, Bili, GGT, sex | 7 | 120 | 0.518 | 80.8 | 0.857 |
| US 6,631,330 | A2M, age, Apo, GGT | 4 | 120 | 0.487 | 77.5 | 0.859 |

## Claims

1. A method for the detection of the presence and/or the severity of a liver disease in a sample from a patient comprising
a) measuring TIMP-1 (Tissue Inhibitor of Metalloproteinase I) in said sample
b) measuring Ferritin in said sample
c) measuring one additional parameter selected from the group consisting of A2M (alpha-2-macroglobulin) and PI (prothrombin index) in said sample
d) diagnosing the presence and/or severity of a liver disease based on the presence or measured levels of TIMP-1, Ferritin and the parameter measured according to step c).

2. Method according to claim 1 used for monitoring therapeutic treatment of liver fibrosis.

3. Method according to claim 1 used for staging liver fibrosis.

## Patentansprüche

1. Verfahren zum Nachweis des Vorhandenseins und/oder der Bestimmung des Schweregrades einer Leberkrankheit in einer Probe aus einem Patienten, bei dem man
a) TIMP-1 (Tissue Inhibitor of Metalloproteinase I) in dieser Probe mißt,
b) Ferritin in dieser Probe mißt,
c) einen zusätzlichen Parameter ausgewählt aus der Gruppe bestehend aus A2M (alpha-2-Makroglobulin) und PI (Prothrombin-Index) in dieser Probe mißt,
d) das Vorhandensein und/oder den Schweregrad einer Leberkrankheit auf Basis des Vorhandenseins oder der gemessenen Konzentrationen an TIMP-1, Ferritin und dem gemäß Schritt c) gemessenen Parameter diagnostiziert.

2. Verfahren nach Anspruch 1 zur Beobachtung der therapeutischen Behandlung von Leberfibrose.

3. Verfahren nach Anspruch 1 zum Staging von Leberfibrose.

## Revendications

1. Procédé pour la détection de la présence et/ou de la gravité d'une maladie hépatique dans un échantillon prélevé d'un patient, comprenant
a) la mesure du TIMP-1 (inhibiteur tissulaire de la métalloprotéinase 1) dans ledit échantillon ;
b) la mesure de la teneur dudit échantillon en ferritine ;
c) la mesure d'un paramètre supplémentaire choisi parmi le groupe constitué par A2M (l'alpha-2-macroglobuline) et PT (le taux de prothrombine) dans ledit échantillon ;
d) le diagnostic de la présence et/ou de la gravité d'une maladie hépatique en se basant sur la présence ou sur des taux mesurés du TIMP-1, de la ferritine et du paramètre mesuré conformément à l'étape c).

2. Procédé selon la revendication 1, utilisé pour la surveillance d'un traitement thérapeutique de la fibrose hépatique.

3. Procédé selon la revendication 1, utilisé pour établir le degré d'une fibrose hépatique.
